# EUROPEAN PATENT SPECIFICATION

(11) **EP 1 082 157 B1**
(45) Date of publication and mention of the grant of the patent: **03.12.2003**
(21) Application number: 99942605.9
(22) Date of filing: 22.03.1999
(51) Int. Cl.: A61M 15/08, B05B 5/00

(54) **NASAL SPRAY DEVICE WITH IMPROVED SPRAY GEOMETRY**
NASENSPRAY MIT VERBESSERTER SPRÜHGEOMETRIE
DISPOSITIF DE PULVERISATION NASALE A GEOMETRIE DE PULVERISATION AMELIOREE

(30) Priority: 31.03.1998 GB 9806939
(43) Date of publication of application: 14.03.2001
(73) Proprietor: THE PROCTER & GAMBLE COMPANY, Cincinnati, Ohio 45202 (US)
(72) Inventor: RENNIE, Paul John, Godalming, Surrey GU7 2HA (GB)
(74) Representative: Clemo, Nicholas Graham
(86) International application number: IB9900469
(87) International publication number: WO99049923

(56) References cited:
- EP-A- 0 501 725
- WO-A-96/40441
- FR-A- 1 146 256
- GB-A- 2 273 673
- US-A- 1 599 111

## Description

### Field of the Invention

The present invention relates to a spray device, particularly to a device adapted to produce a spray comprising a fluid ligament with a spray cone diverging from one end of the ligament. More particularly, the invention relates to an electrostatic spray device capable of delivering a soft spray to the nasal cavity without needing to be inserted into the nostrils. The disclosure shows also a method of electrostatically spraying a liquid in such a way that the liquid is initially projected from a spray head in the form of a ligament which enters the nostrils and thereafter breaks up into droplets under the influence of Coulombic forces to produce an atomised spray.

### Background of the Invention

Treatment of maladies affecting the nasal region, such as hay fever or congestion due to colds, has long been effected by means of a nasal spray device. More recently it has been recognised that the mucous membranes of the nasal cavity can be used as a convenient delivery site for drugs targeted at other areas of the body. See for example WO 92/11049 which discloses a pen shaped device for nasal administration of, particularly, insulin. Invariably, however, devices for administration of fluids into the nose have been designed such that the device must be partially inserted into the nostrils to be effective. Not only is the manner of using such a device potentially unhygienic and/or uncomfortable for the user, it is often required that the user tilt his or head backwards to avoid the fluid running out of the nose once dispensed.

The nasal cavity is a difficult area of the body to target with drugs. To reach the inner nasal cavity, the drug must be introduced into the vestibule of the nose via the nostrils, travel to the back of this cavity and then pass through the narrow slit-shaped opening of the nasal valve. Once through the nasal valve, the drug then needs to be distributed onto the vascular tissues of the inferior and middle turbinates. The turbinates are finger-like projections of erectile tissue covered with ciliated epithelium and goblet cells. When the nose becomes congested, the turbinates expand and close off the air passages. To be effective, topical drugs such as decongestants, antiinflammatories, or antihistamines must coat the epithelium of the turbinates, where they can exert their pharmacological action. The turbinates are the most highly vascularised area of the nose, and therefore are the preferred point of absorption of systemic drugs.

It has now been found that, by designing a spray with a particular ligament length and a suitably dimensioned spray cone diverging from one end of the ligament, a spray can be targeted to a suitable part of the nose, especially the turbinates, for absorption of an active, without the device needing to be inserted into the nostrils.

A preferred means of generating the spray is by use of an electrostatic device. An electrostatic device for nasal or oral delivery of a fluid is described in WO 96/40441. Ligament mode electrostatic spray devices are also described in EP-A-501,725 and GB-A-2 273 673. Copending application PCT/GB97/02746 describes a device suitable for electrostatic spraying of low-resistivity fluids such as aqueous fluids.

The aforesaid WO 96/40441 describes at p9, 19 - 21 that its device will be used by inserting the nose-piece into the nostril and spraying whilst the user inhales. Accordingly it suffers from the same limitation as most prior art devices that, to be effective, insertion of the device into the nostrils is required. This can cause discomfort and is perceived by many as being unhygienic, leading to inhibition of using the device. Spraying from outside the nostrils, however, often leads to inefficient delivery of the spray to the intended target, especially the turbinates of the nose, since much of the fluid falls on the outside of the intended target and gives rise to uncomfortable 'run-off'.

It has now been found that a spray device can be designed which overcomes these problems.

Accordingly, it is an object of the present invention to provide a spray device which has improved effectiveness and /or comfort in delivering fluids to the nasal cavity.

It is a further object of this invention to provide a spray device for nasal administration of a fluid without substantial penetration of the device into the nostrils.

It is yet a further object of this invention to provide a spray device which delivers a soft gentle spray for greater user comfort.

It is yet a further object of this invention to provide a spray device which efficiently delivers a low unit volume.

It is yet a further object of this invention to provide a nasal spray device which delivers a fluid without causing a wet sensation in the nasal vestibule or on the nostrils.

### Summary of the Invention

According to the present invention there is provided a spray device as defined in claim 1.

### Detailed Description of the Invention

### Fluids

The spray device of the invention comprises a fluid reservoir containing a pharmaceutically acceptable fluid, the fluid comprising a pharmaceutically acceptable treatment agent selected from medicaments, flavours, salts, surfactants and mixtures thereof. The fluid optionally comprises other adjuvants dissolved or dispersed within it. The fluid can be aqueous or non-aqueous. Suitable aqueous fluids include water and mixtures of water with water-miscible solvents such as glycerol, propylene glycol, or alcohols such as ethanol or isopropyl alcohol. Aqueous emulsions can also be used, either water-in-oil or oil-in water emulsions. Preferably the fluid is an aqueous solution, dispersion or oil-in-water emulsion. Suitable non-aqueous fluids comprise polyethylene glycols, glycerol, propylene glycol, dimethyl isosorbide, silicone oils, ketones, ethers and mixtures thereof.

Although not limited to any particular range of resistivity, the invention has particular application to low resistivity fluids, especially those having a bulk resistivity of less than 1 x 10⁸ ohm.cm, preferably those having a resistivity of less than 1 x 10⁴ ohm.cm, more preferably less than 1 x 10³ ohm.cm. If necessary, the fluid may comprise a resistivity modifier, such as a pharmaceutically acceptable salt, in order to bring the bulk resistivity within the required range.

The fluid is preferably a pharmaceutically acceptable intranasal carrier. Preferably, the nasal composition is isotonic, i.e., it has the same osmotic pressure as blood and lacrimal fluid. The desired isotonicity of the compositions of this invention may be accomplished using, for example, the sodium chloride already present, or other pharmaceutically-acceptable agents such as dextrose, boric acid, citric acid, sodium tartrate, sodium citrate, sodium phosphate, potassium phosphate, propylene glycol or other inorganic or organic solutes. Sodium chloride is preferred particularly for buffers containing sodium ions. Further examples of sodium chloride equivalents are disclosed in Remington's Pharmaceutical Sciences pp. 1491-1497 (Alfonso Gennaro 18th ed. 1990).

### Medicaments

The fluid can comprise a wide range of medicaments. By "medicament" is meant a drug or other substance intended to have a therapeutic effect on the body. Suitable levels of the medicament are from 0.001 to 20%, preferably from 0.01 to 5%, more preferably from 0.1 to 5%. It will be appreciated that the levels of specific medicaments will depend on many factors including their potency, safety profile, solubility / ease of dispersion and intended effect. The medicament, when used, can be one which is intended to have an effect at the site of application, such as a decongestant, antihistamine or anti-inflammatory drug, or it may be intended for systemic absorption such as an anti-viral, anti-depressant, anti-emetic, anti-pyretic medicament or a hormone or such-like. The medicament can be soluble in the fluid or can be an insoluble, finely divided particulate liquid or solid dispersed within the fluid.

Suitable decongestants include oxymetazoline, tramazoline, xylometazoline, naphazoline, tetrahydrazoline, pseudoephedrine, ephedrine, phenylephrine, their pharmaceutically acceptable salts, such as the hydrochlorides, and mixtures thereof. Preferred decongestants are selected from oxymetazoline, xylometazoline, their pharmaceutically acceptable salts and mixtures thereof Especially preferred for use herein is oxymetazoline hydrochloride which is soluble in water. When used in the compositions of the present invention, the decongestant is preferably present at a concentration of from about 0.01% to about 3.0%, more preferably from about 0.01 % to about 1%.

Antihistamines useful to the present invention include, but are not limited to, fast-acting, histamine H-1 receptor antagonists. Such H-1 receptor antihistamines may be selected from among the following groups of antihistamines: alkylamines, ethanolamines, ethylenediamines, piperazines, phenothiazines, piperidines. Examples of useful fast acting antihistamines include acrivastine, carbinoxamine, diphenhydramine, chloropheniramine, brompheniramine, dexchloropheniramine, doxylamine, clemastine, promethazine, trimeprazine, methdilazine, hydroxyzine, pyrilamine, tripelennamine, meclizine, triprolidine, azatadine, cyproheptadine, rocastine, phenindamine or pharmaceutically acceptable salts and mixtures thereof. Other useful antihistamines include terfenadine, azelastine, cetirizine, astemizole, ebastine, ketotifen, lodoxamide, loratadine, levocabastine, mequitazine, oxatomide, setastine, tazifylline, temelastine or pharmaceutically acceptable salts and mixtures thereof When used in the compositions of the present invention, the antihistamine component is preferably present at a concentration of from about 0.01% to about 3.0%, more preferably from about 0.01% to about 1%.

The medicament can also be an anti-inflammatory agent such as a corticosteroid. Particularly preferred agents within this class are glucocorticoids selected from the group consisting of beclomethasone, flunisolide, fluticasone, memetasone, budesonide, pharmaceutically acceptable salts thereof and mixtures thereof When used in the compositions of the present invention, the anti-inflammatory agent is preferably present at a concentration of from about 0.001% to about 0.1%, more preferably from about 0.01 % to about 0.1 %.

Also useful herein are xanthine derivatives such as caffeine and methylxanthine and the like; antiallergics; mucolytics; anticholinergics; non-opiate analgesics such as acetaminophen, acetylsalicylic acid, ibuprofen, etodolac, fenbuprofen, fenoprofen, ketorolac, flurbiprofen, indomethacin, ketoprofen, naproxen, pharmaceutically acceptable salts thereof and mixtures thereof; opiate analgesics such as butorphanol; leukotriene receptor antagonists; mast cell stabilisers such as cromolyn sodium, nedocromil and lodoxamide; and lipoxygenase inhibiting compounds.

Further examples of suitable medicaments can be found in WO97/46243 EP-A-780127, US-A-5,124,315, US-A-5,622,724, US-A-5,656,255 and US-A-5,705,490

### Flavours

Various flavouring and/or aromatic components (e.g., aldehydes and esters) can be used in the fluids of the invention. These include, for example, menthol, camphor, eucalyptol, benzaldehyde (cherry, almond); citral (lemon, lime); neral; decanal (orange, lemon); aldehyde C-8, aldehyde C-9 and aldehyde C-12 (citrus fruits); tolyl aldehyde (cherry, almond); 2,6-dimethyl-octanal (green fruit); 2-dodecenal (citrus, mandarin); and herbal components such as thyme, rosemary and sage oils. Additional aromatic components suitable for use in the present invention include those described in U.S. Patent 4,136,163 to Watson et al., U.S. Patent 4,459,425 to Amano et al., and U.S. Patent 4,230,688 to Rowsell et al.. Mixtures of these aromatics can also be used.

### Surfactants

The fluid can also comprise one or more pharmaceutically acceptable surfactants. Such surfactants can be useful for dispersing or emulsifying medicaments or flavours, for enhancing absorption across the nasal membrane or as treatment agents in their own right, such as for softening earwax. The surfactants can be anionic, nonionic, cationic or amphoteric, preferably they are nonionic. Typical nonionic surfactants useful herein include: polyoxyethylene derivatives of fatty acid partial esters of sorbitol anhydrides such as polysorbate 80; polyoxyethylene derivatives of fatty acids such as polyoxyethylene 50 stearate, as well as oxyethylated tertiary octyl phenol formaldehyde polymer (available from Sterling Organics as Tyloxapol) or mixtures thereof. The usual concentration is from 0.1% to 3% by weight.

### Salts

The fluid can also comprise one or more pharmaceutically acceptable salts. The salt can mineral salts such as e.g. sodium chloride, or salts of organic acids such as sodium citrate.

### Other adjuvants

The fluid can further comprise other ingredients such as thickeners, humectants, suspending aids, encapsulating aids, chelating agents and preservatives.

The viscosity of the compositions may be maintained at a selected level using a pharmaceutically-acceptable thickening agent. Suitable thickening agents include, for example, xanthan gum, methyl cellulose, microcrystalline cellulose, carboxymethyl cellulose, chitosan, hydroxypropyl cellulose, hydroxypropyl methyl cellulose, hydroxymethyl cellulose, hydroxyethyl cellulose, carboxyvinyl polymer, carbomer, and the like or pharmaceutically acceptable salts thereof Mixtures of such thickening agents may also be used. The preferred concentration of the thickener will depend upon the agent selected. The important point is to use an amount which will achieve the selected viscosity. Viscous compositions are normally prepared from solutions by the addition of such thickening agents.

Fluids useful in the present invention can also comprise from about 0.01% to about 5% of a humectant to inhibit drying of the mucous membrane and to prevent irritation. Any of a variety of pharmaceutically-acceptable humectants can be employed including, for example sorbitol, propylene glycol, polyethylene glycol, glycerol or mixtures thereof. As with the thickeners, the concentration will vary with the selected agent, although the presence or absence of these agents, or their concentration is not an essential feature of the invention.

A pharmaceutically-acceptable preservative is generally employed to increase the shelf life of the compositions of the present invention. A variety of preservatives including, for example, benzyl alcohol, parabens, phenylethyl alcohol, thimerosal, chlorobutanol, chlorhexidine gluconate, or benzalkonium chloride can be employed. The most preferred preservative system for use herein comprises a combination of benzalkonium chloride, chlorhexidine gluconate and disodium EDTA as a chelating agent. A suitable concentration of the preservative will be from 0.001 % to 2% based on the total weight, although there may be appreciable variation depending upon the agent selected.

### Spray devices and spray characteristics

The spray device will generally comprise a means for controlling the dosage of the dispensed fluid. This can be as simple as an on-off switch which allows the user to control the dosage according to his or her needs. Preferably the spray device is adapted to provide a unit fluid dose, more particularly a unit dose with a volume in the range of from about 1 to about 100 µl, preferably from about 1 to about 20, more preferably from about 5 to about 15 µl. In especially preferred embodiments the device is adapted to provide multiple unit doses of the volumes mentioned. The dose volume is preferably pre-set but it can also be adjusted by the user to a desired volume. The device suitably comprises a reservoir for holding the fluid and a dosing means for selectively delivering a unit dose from the reservoir to the spray generator.

The dosing means can be, for example, a metered valve or a syringe pump.

The device is adapted to produce a spray having a fluid ligament, the ligament extending from the nosepiece and having a nosepiece end and a delivery end, the spray further comprising a spray cone diverging from the delivery end of the ligament. By "nosepiece end" is meant the point at which a plane (hereinafter the nosepiece plane) drawn perpendicular to the axis of the ligament and just touching the exterior of the nosepiece would intersect the centre of the ligament. The ligament has a length of from about 1 to about 20 mm, preferably from about 1 to about 10 mm, more preferably from about 2 to about 8 mm, and especially from about 3 to about 6mm from the nosepiece end to the delivery end.

In preferred embodiments the spray cone has a cone angle of from about 10 to about 90°, preferably 20 to about 50°, more preferably from about 30 to about 40°. In electrostatic devices, the length of the ligament and the spray cone angle can be adjusted by varying the viscosity or surface tension of the fluid, by varying the fluid flow rate or exit velocity, or by varying the electrical field strength through applied voltage, potential gradient or by use of a field intensifying electrode.

The total length of the ligament is longer than the length from the nosepiece end to the delivery end since the ligament originates from a point on the device side of the nosepiece plane, such as from an elastomeric, self-sealing valve as disclosed herein, and passes through a passage in the nosepiece. Suitably the distance from the point of origin of the ligament to the nosepiece plane is in the range from about 2 mm to about 15 mm, preferably from about 3 to about 10 mm and more preferably from about 5 to about 9 mm. In this way the nosepiece can be employed as a field intensifier which helps to control the ligament length. For this purpose the nosepiece is preferably a non-conducting material such as a plastic which can be e.g. polypropylene but is preferably a soft thermoplastic elastomer which provides for greater comfort if held against the nose. Elastomers described herein for the self-sealing valve are also suitable for the nosepiece.

Electrostatic devices suitable for ligament mode spraying are described in WO 96/40441, in EP-A-501,725 and in co-pending application PCT/GB97/02746. Preferably the present device is a device according to embodiments of EP-A-501,725 or PCT/GB97/02746 in which a jet is created by mechanical means and a high voltage applied to the fluid leads to the jet or ligament breaking up into a spray cone. The jet can be generated by, for example, a syringe pump. Suitable jet velocities are from about 0.5 to about 8, preferably from about 1 to about 3 ms⁻¹.

A suitable high voltage is in the range from about 1 kV up to about 10 kV, preferably from about 2kV to about 7kV and more preferably from about 2kV to about 5kV.

The voltage can conveniently be applied to the fluid, even within the constraints of a small hand-held device, from a low voltage (1.5V is sufficient) battery coupled to a step-up transformer. The battery is preferably of the long-life type and can be rechargeable. If a metal syringe pump is used, the voltage can be applied to the fluid through the pump which is preferably encased in an insulating plastic housing. Alternatively, the fluid can be charged by an electrode inserted into the fluid. The generator can be activated by the user by means of an external switch which can also be used to mechanically prime the pump. Preferably the switch includes a metal portion by means of which the user completes an earth return path to the high voltage circuit. A suitable arrangement for the overall device construction is described in PCT/GB97/02746. In this way the user does not acquire a net charge. Alternate arrangements, whereby an alternating voltage is applied, can also be used to prevent charge build-up.

The device is activated to deliver the spray. The ligament of the spray extends through the nostril opening, into the vestibule and preferably to within a short distance of the nasal valve opening, before breaking up to form the spray cone. The device can be constructed simultaneously to dispense two sprays, directed to each of two nostrils. The two sprays can be generated from two separate dosing means or can be provided from a single source such as by using a 'Y' shaped valve to split a single jet into two.

In order to provide clean cut-off at low unit volumes the device preferably comprises an elastomeric, self-sealing exit valve having a fluid side and a delivery side, the valve opening to allow passage of the fluid when pressure is applied to fluid on the fluid side and sealing when the pressure is removed. By "exit valve" is meant that the elastomeric valve is the final dispensing valve and that there are no other elements of the device which mechanically, restrict or modify the flow of the fluid on the downstream side of the valve. In highly preferred embodiments herein the valve is a slit valve. The valve can comprise a single slit or tow or more intersecting slits, to form a cross shape for example. Preferably, however, the valve comprises a single slit. Although the valve can be flat it is preferably dome-shaped by which is meant that a non-planar valve having a recess such as with a hemispherical or frustoconical dome. In preferred embodiments the valve is essentially in the form of a hemispherical dome having a flange along its perimeter so that a collar can be fitted to retain the valve in the device. The diameter of the valve, including the flange, is typically from about 2 to about 6 mm with the dome portion having a diameter of from about 1 to about 4 mm, typically about 2.5mm and a thickness from inside to out of from about 0.5 to about 1.5 mm, suitably about 1 mm. The valve need not be of uniform thickness. In preferred embodiments the valve dome's exterior surface is hemispherical whereas the internal surface is formed with a small flat at the top of the dome where the slit is formed. Suitable slit widths are from about 50 to about 400 µm, preferably from about 150 to about 250 µm. It is to be understood that the slit width refers to the longest dimension of the slit when first created. The term "elastomer" herein refers to a material which is both elastically compressible and elastically extensible. A wide range of elastomers can be used, including but not limited to polyurethanes; chloroprene, butyl, butadiene and styrene-butadiene rubbers, and silicone elastomers such as 2 part room temperature vulcanising (RTV) silicones. Preferred for use herein are the 2 part silicone RTVs. Suitable silicone RTVs are available under the trademark NuSil and have a hardness of from about 30 to about 80 Shore A, preferably from about 40 to about 70 Shore A. The elastomers can optionally be mixed with a suitable plasticiser or foaming agent to make them more compressible. The elastomer may also have other materials dispersed within it in order to modify the its properties, such as its conductivity. If elastomers of low tear strength are employed, the slit width may grow if the slit is held open for a prolonged period of time. The slit valve can be formed by piercing an injection moulded elastomeric seal, of the same dimensions and shape as the intended valve, with a pin having a sharpened tip. The slit width is roughly proportional to the pin width. The pin can be a flat blade or can have a polygonal or round cross-section. The pin preferably has a polygonal or, especially, a round cross-section. It has been found that sharp-edged pins create a cut which behaves in use as a flap rather than a hole. This can lead to a jet which is not straight or even to the creation of two or more jets which may lead to unreliable or unpredictable spraying. Suitable pin diameters are from about 100 to 350, more preferably from 150 to 250 µm in diameter. When silicone elastomers are used it is preferred that the piercing pin is withdrawn rapidly after forming the slit to avoid undesired slit growth. It has further been found that the geometry of the elastomeric seal and the method of piercing have a significant effect on the effectiveness and reproducibility of slit formation. More reliable slit formation is obtained if the seal is pierced from the inside of the dome rather than from the outside.

The valve opens when pressure is applied to fluid on the fluid side and seals when the pressure is removed. As mentioned earlier, pressure can be applied to the fluid by of a dosing means such as a syringe pump. The applied pressure is suitably in the range from about 200 to about 5000 mbar (20 to 500 kPa), preferably from about 500 to about 3000 mbar (50 to 300 kPa). The flow rate through the valve is generally proportional to the pressure applied and is suitably in the range from about 5 to about 50, more preferably from about 5 to about 30 µls⁻¹. At such pressures and with the disclosed valve type and slit dimensions a straight fluid ligament with an exit velocity of from about 0.5 to about 8 preferably from about 1 to about 3 ms⁻¹ is obtained.

The diameter of the issuing ligament is partly determined by the flow rate and is generally less than the width of the slit valve. Depending on the flow rate, ligament diameters of less than 50 µm can be achieved, even when a valve slit width of 200 µm is employed. The ligament diameter strongly influences the particle size of the spray after break-up into the spray cone, the particle size being broadly similar to the ligament diameter. It is a feature of the ligament mode electrostatic spraying herein that a tightly distributed, almost mono-disperse spray is obtained. It is thus possible to achieve a spray with a median droplet size of from 20 to about 80, preferably from about 30 to about 70 µm, more preferably from about 40 to about 60 µm, the particle size distribution generally having a standard deviation of less than 5, typically less than 2 µm, and preferably less than 1 µm. It is commonly understood that, for nasal spraying, a particle size of 10 µm or less is desirable so that the particles are not carried through into the lungs. It is believed, however, that having an electrostatic charge on the spray particles makes them much less likely to be carried beyond the nose since the charged particles tend to find an earthed surface rather quickly.

The clean stop performance of the tip valve can be further improved by introducing a pressure relief feature behind the valve. This would take the form of a by-pass to the fluid reservoir so that any residual fluid pressurised by relaxation of the elastomer would return to a reservoir rather than dripping from the valve exit.

If necessary, several self-sealing valves can be used in tandem to achieve higher volume throughputs whilst retaining the advantageously small spray particle sizes. The seals from which the valves are made can conveniently be manufactured by a conventional injection moulding process.

### Methods

The spray device herein is suitable for spraying into a bodily cavity, particularly into the nose, mouth or ears of a human. The low volume and gentle spray also make it suitable for e.g. ophthalmic spraying. Preferably the device is a nasal spray device. A preferred method of administering a fluid to the nasal cavity from the spray device comprises spraying the fluid into the nasal cavity without substantial penetration of the device into the nostrils. By "without substantial penetration into the nostrils" herein is meant that there is no insertion of a nozzle or such-like into the nasal vestibule. In use, the nosepiece of the device is preferably placed in contact with the nostril opening to obtain the full benefit of the field intensifying effect described herein in relation to the nosepiece. If pressure is applied by the user, for certainty of contact or to assist in orientation there may be some flaring of the nostril or overlap with the septum cartilage but nevertheless the nosepiece will not be completely surrounded by the nostril.

The invention will now be described by way of example only, with reference to the accompanying drawings in which:
- Fig.: 1 is a sectional view through a human nose.
- Fig. 2: is a perspective view of a spray device according to the invention.
- Fig. 3: is a greatly simplified sectional view of the spray device of Figure 2 showing the relationship of the elastomeric nozzle to the nosepiece and dosing means.
- Fig. 4: is a schematic part section showing a spray issuing from a device according to the invention.
- Fig. 5: is a enlarged sectional view of an elastomeric exit valve.

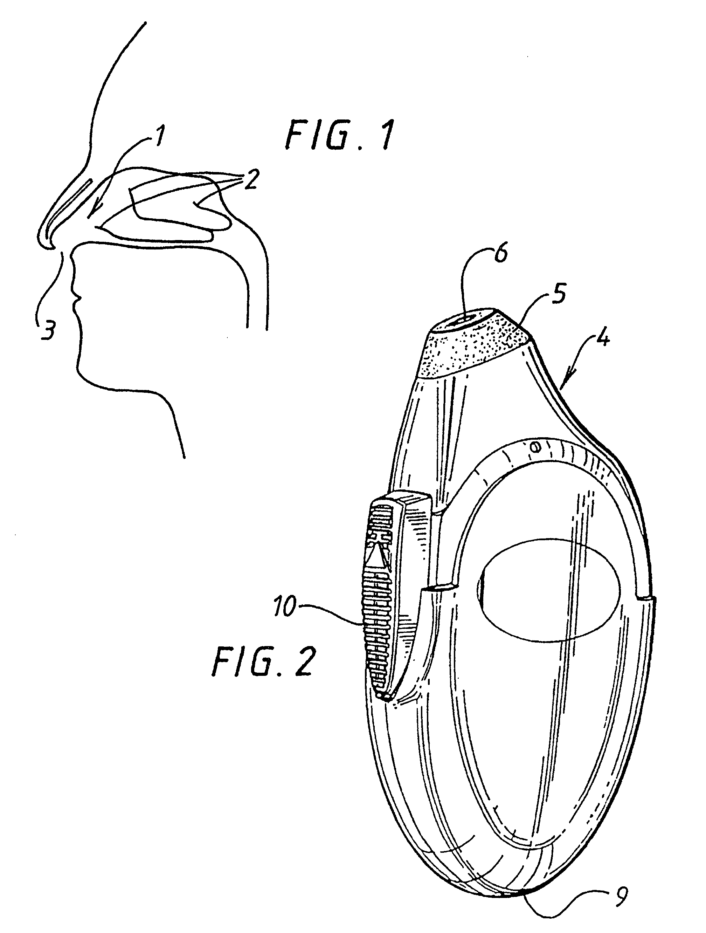

Referring to Fig. 1, the nose comprises a nasal cavity 1 in which are located the turbinates 2, convoluted finger-like structures covered with epithelium and which are susceptible to inflammation. The exterior opening to the nasal cavity is through two nostrils 3. Since the turbinates are located in the posterior part of the nasal cavity it has generally been difficult to spray them effectively without inserting a spray device into the nostrils.

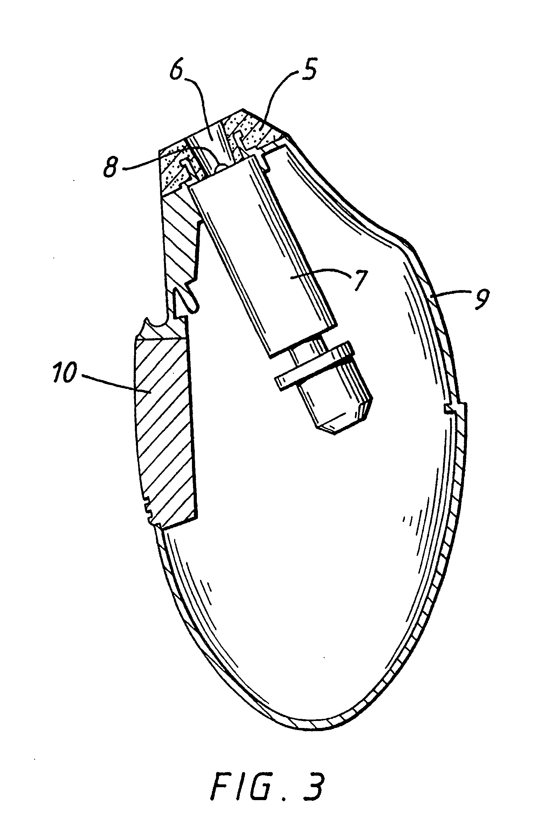

Referring to Figures 2 and 3, there is shown a spray device 4 fitted with a soft elastomeric nosepiece 5 which is designed to fit against a user's nostril opening without being inserted into the nostril. The nosepiece is provided with a passage 6 which connects to a syringe pump 7, the interior detail of which is not shown, which is used to generate the ligament of the spray through elastomeric exit valve 8. The insulating casing 9 of the device further encloses (not shown) a replaceable fluid reservoir, battery, transformer and electronic circuitry for supplying a high voltage from the transformer to the fluid. The device is supplied with an actuating mechanism 10 which mechanically primes the pump and triggers its release to deliver a pre-set unit dose of the fluid. The actuating mechanism includes a metal portion to provide an earth return from the user to the high voltage circuitry.

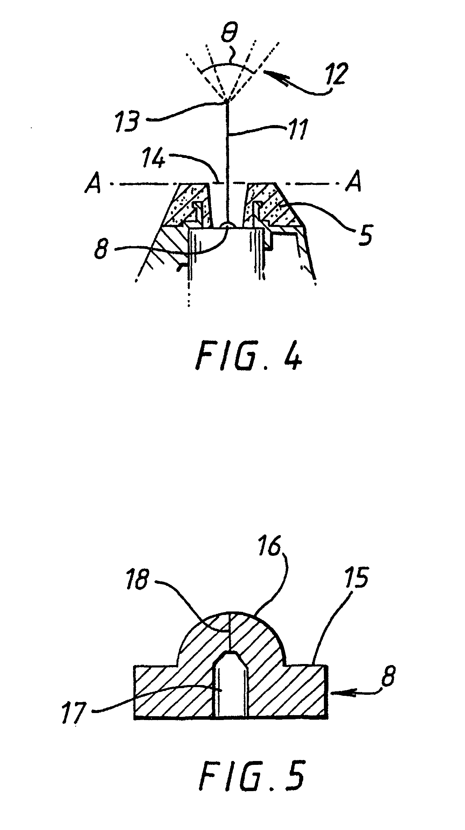

Referring now to Figure 4, a spray comprises ligament 11 and spray cone 12 having a cone angle θ. In practice the cone angle can be measured by spraying from a fixed distance onto disclosing paper and then measuring the diameter of the spray pattern or by direct viewing of the spray using high speed video photography with back lighting. The ligament has a delivery end 13 where the ligament breaks up into the spray cone and a nosepiece end 14 defined as the point where plane A-A intersects ligament 11. Plane A-A is drawn perpendicular to the axis of the ligament, just touching the tip of nosepiece 5.

Figure 5 illustrates an elastomeric exit valve 8. The nozzle, which is circularly symmetrical, comprises a flange 15 for securing the nozzle to the spray device, a domed head 16, a recess 17 and a slit 18 extending from the recess to the top of the dome. The nozzle is formed by injection moulding a seal of essentially the same construction and then piercing the dome with a sharpened pin, of round cross-section and 200 µm diameter, from the inside of the recess.

### Examples

The following examples further describe and demonstrate embodiments within the scope of the present invention. The examples are given solely for the purpose of illustration and are not to be construed as limitations of the present invention.

### Example I

A fluid of the present invention is prepared by combining the following components utilising conventional mixing techniques similar to that described below.

| **Component** | **Wt %** |
|---|---|
| oxymetazoline hydrochloride | 0.31 |
| sodium citrate dihydrate | 1.75 |
| citric acid | 0.35 |
| Tyloxapol | 0.70 |
| chlorhexidine digluconate | 0.054 |
| benzalkonium chloride | 0.02 |
| camphor | 0.04 |
| eucalyptol | 0.02 |
| disodium EDTA dihydrate | 0.01 |
| distilled water | q.s. 100ml |

In an appropriately sized vessel, the above listed ingredients are added one at a time to water with mixing, allowing each to dissolve before adding the next. After all the ingredients have been added, purified water is used to bring the batch to the appropriate weight. The solution has a bulk resistivity of 120 ohm.cm. The solution is charged into a flexible laminate reservoir and fitted into an electrostatic spray device of the kind indicated in figure 2. The nosepiece of the device is held against the nostril and the device directed such that the spray ligament will enter the nostril. On actuation of the device, 8 µl of the solution is dispensed over a period of about 1 second. The dispensed fluid provides relief from nasal decongestion without causing noticeable wetness either on or inside the nose. The low dose volume and gentle delivery have the result that the user does not sense any appreciable physical impact from the spray.

### Example II

A further fluid is prepared by combining the following components utilising conventional mixing techniques similar to that described in Example I.

| **Component** | **Wgt %** |
|---|---|
| flunisolide | 0.025 |
| chlorpheniramine | 0.350 |
| levocabastine | 0.0125 |
| propylene glycol | 2.000 |
| polyethylene glycol | 1.000 |
| ethylenediamine tetraacetic acid | 0.050 |
| benzalkonium chloride | 0.010 |
| distilled water | q.s. 100ml |

Intranasal spray administration, in the manner of Example I, of approximately 10 µl of the fluid composition is used to provide relief from allergy or allergy-like symptoms.

### Example III

A fluid of the present invention is prepared by combining the following components utilising conventional mixing techniques similar to that described in Example I.

| **Component** | **Wt %** |
|---|---|
| beclomethasone diproprionate, monohydrate | 0.042 |
| chlorpheniramine | 0. 500 |
| Avicel RC - 591¹ | 0.200 |
| dextrose | 5.100 |
| polysorbate 80 | 0.050 |
| benzalkonium chloride | 0.020 |
| phenylethyl alcohol | 0.025 |
| distilled water | q.s. 100ml |

| | |
|---|---|
| ¹microcrystalline cellulose and sodium carboxymethyl cellulose, supplied FMC Corporation. | |

Intranasal spray administration, in the manner of Example I, of approximately 10 µl of the fluid composition is used to provide relief from allergy or allergy-like symptoms.

## Claims

1. An electrostatic spray device (4) comprising a spray generator, a fluid reservoir and a nosepiece (5) having an external tip and a passage (6) extending therethrough wherein:
i) the fluid reservoir contains a pharmaceutically acceptable fluid, the fluid comprising a pharmaceutically acceptable treatment agent selected from medicaments, flavours, salts, surfactants and mixtures thereof; and
ii) the device is adapted to produce a spray having a fluid ligament (11) extending through the passage (6) in the nosepiece (5) and a high voltage applied to the fluid leads to the ligament breaking up into a spray cone (12), the ligament having a nosepiece end (14) at the intersection between the ligament and a plane perpendicular to it and the external tip of the nosepiece and a delivery end (13), wherein the spray cone diverges from the delivery end (13) of the ligament;
**characterised in that**
the ligament has a velocity of from 0.5 to 8 ms⁻¹ and a length of from 1 to 20 mm from the nosepiece end (14) to the delivery end (13) whereby the device is suitable for spraying into the nostrils (3) of a user to target the nasal turbinates (2).

2. An electrostatic spray device according to Claim 1 wherein a voltage in the range from 1 kV up to 10 kV is applied to the fluid.

3. A spray device according to Claim 1 or Claim 2 wherein the spray cone has a cone angle of from 10 to 90°, preferably 20 to 50°, more preferably from 30 to 40°.

4. A spray device according to any of Claims 1 to 3 wherein the device is adapted to provide a unit fluid dose with a volume in the range from 1 to 20, preferably from 5 to 15 µl.

5. A spray device according to Claim 4 wherein the device is adapted to provide multiple unit fluid doses.

6. A spray device according to any preceding claim wherein the fluid reservoir is replaceable.

## Patentansprüche

1. Elektrostatische Sprühvorrichtung (4), umfassend einen Sprühgenerator, ein Flüssigkeitsreservoir und ein Nasenstück (5), mit einer externen Spitze und einem Durchgang (6), der sich durch dieses erstreckt, wobei:
i) das Flüssigkeitsreservoir eine pharmazeutisch annehmbare Flüssigkeit enthält, die Flüssigkeit ein pharmazeutisch annehmbares Behandlungsmittel umfasst, ausgewählt aus Medikamenten, Geschmacksstoffen, Salzen, Tensiden und Mischungen daraus; und
ii) die Vorrichtung so angepasst ist, dass ein Sprühnebel erzeugt wird, der ein Flüssigkeitsband (11) aufweist, welches sich durch den Durchgang (6) in das Nasenstück (5) erstreckt, und wobei eine an die Flüssigkeit angelegte Hochspannung dazu führt, dass das Band in einem Sprühnebelkonus (12) zerfällt, wobei das Band ein Nasenstückende (14) am Schnittpunkt zwischen dem Band und einer Ebene, welche dazu lotrecht ist und die externe Spitze des Nasenstücks berührt, und ein Zulieferungsende (13) aufweist, wobei der Sprühnebelkonus aus dem Zulieferungsende (13) des Bandes divergiert;
**dadurch gekennzeichnet, dass**
das Band eine Geschwindigkeit von 0,5 bis 8 m/s und eine Länge von 1 bis 20 mm besitzt, vom Nasenstückende (14) bis zum Zulieferungsende (13), wobei die Vorrichtung dazu geeignet ist, in die Nasenlöcher (3) eines Anwenders zu sprühen, um die Nasenmuscheln (2) zu erreichen.

2. Elektrostatische Sprühvorrichtung nach Anspruch 1, wobei eine Spannung im Bereich von 1 kV bis zu 10 kV an die Flüssigkeit angelegt wird.

3. Sprühvorrichtung nach Anspruch 1 oder 2, wobei der Sprühkonus einen Konuswinkel von 10 bis 90° besitzt, vorzugsweise 20 bis 50°, noch bevorzugter von 30 bis 40°.

4. Sprühvorrichtung nach irgendeinem der Ansprüche 1 bis 3, wobei die Vorrichtung so ausgelegt ist, dass eine Flüssigkeitsdosiseinheit mit einem Volumen im Bereich von 1 bis 20, vorzugsweise 5 bis 15 µl vorgesehen wird.

5. Sprühvorrichtung nach Anspruch 4, wobei die Vorrichtung so ausgelegt ist, dass mehrfache Flüssigkeitsdosiseinheiten vorgesehen werden.

6. Sprühvorrichtung nach irgendeinem der vorangehenden Ansprüche, wobei das Flüssigkeitsreservoir austauschbar ist.

## Revendications

1. Dispositif de pulvérisation électrostatique (4) comprenant un générateur de pulvérisation, un réservoir de fluide et une pièce nasale (5) présentant une pointe externe et un passage (6) s'étendant à travers celle-ci, dans lequel :
i) le réservoir de fluide contient un fluide acceptable du point de vue pharmaceutique, le fluide comprenant un agent de traitement acceptable du point de vue pharmaceutique choisi parmi les médicaments, les substances aromatisantes, les sels, les agents tensioactifs et leurs mélanges ; et
ii) le dispositif est apte à produire une pulvérisation présentant un flux de fluide (11) s'étendant à travers le passage (6) de la pièce nasale (5), et une tension élevée appliquée au fluide fait que le flux se désagrège selon un cône de pulvérisation (12), le flux présentant une extrémité de pièce nasale (14) à l'intersection entre le flux et un plan perpendiculaire à celui-ci et touchant la pointe externe de la pièce nasale, et une extrémité de distribution (13), le cône de pulvérisation divergeant à partir de l'extrémité de distribution (13) du flux ;
**caractérisé en ce que**
le flux a une vitesse de 0,5 à 8 ms⁻¹ et une longueur de 1 à 20 mm de l'extrémité de pièce nasale (14) à l'extrémité de distribution (13), de sorte que le dispositif est approprié pour effectuer des pulvérisations dans les narines (3) d'un utilisateur afin d'atteindre les cornets (2) du nez.

2. Dispositif de pulvérisation électrostatique selon la revendication 1, dans lequel on applique au fluide une tension comprise dans la plage de 1 kV à 10 kV.

3. Dispositif de pulvérisation selon la revendication 1 ou 2, dans lequel le cône de pulvérisation a un angle de cône de 10 à 90°, de préférence, de 20 à 50°, mieux encore, de 30 à 40°.

4. Dispositif de pulvérisation selon l'une quelconque des revendications 1 à 3, dans lequel le dispositif est apte à fournir une dose de fluide unitaire ayant un volume compris dans la plage de 1 à 20, de préférence, de 5 à 15 µl.

5. Dispositif de pulvérisation selon la revendication 4, dans lequel le dispositif est apte à fournir plusieurs doses de fluide unitaires.

6. Dispositif de pulvérisation selon l'une quelconque des revendications précédentes, dans lequel le réservoir de fluide peut être remplacé.
